# EUROPÄISCHE PATENTSCHRIFT

(11) **EP 0 741 565 B1**
(45) Veröffentlichungstag und Bekanntmachung des Hinweises auf die Patenterteilung: **19.04.2000**
(21) Anmeldenummer: 95906207.6
(22) Anmeldetag: 26.01.1995
(51) Int. Cl.: A61K 31/19, A61K 9/14, A61K 9/16

(54) **VERFAHREN ZUR HERSTELLUNG VON S(+)-IBUPROFEN-PARTIKELN**
PROCESS FOR PRODUCING S(+)-IBUPROFEN PARTICLES
PROCEDE DE PRODUCTION DE PARTICULES D'IBUPROFENE S(+)

(30) Priorität: 28.01.1994 AT 15894
(43) Veröffentlichungstag der Anmeldung: 13.11.1996
(73) Patentinhaber: Gebro Pharma GmbH, 6391 Fieberbrunn (AT)
(72) Erfinder: MÖLLER, Torsten, D-83352 Altenmarkt-Alz (DE); HANTICH, Gerhard, A-6370 Kitzbühel (AT); HESSE, Ernst, A-6391 Fieberbrunn (AT)
(74) Vertreter: Brauneiss, Leo, Dipl.Ing.
(86) Internationale Anmeldenummer: AT9500014
(87) Internationale Veröffentlichungsnummer: WO9520382

(56) Entgegenhaltungen:
- EP-A- 0 120 587
- EP-A- 0 299 668
- EP-A- 0 362 728
- EP-A- 0 362 731
- WO-A-90/03782
- WO-A-92/08686
- US-A- 4 086 346
- DATABASE WPI Week 7744, Derwent Publications Ltd., London, GB; AN 77-78258Y (44) & JP,A,52 111 533 (KANEBO KK) 19. September 1977

## Beschreibung

Die Erfindung bezieht sich auf ein Verfahren zur Herstellung von S(+)-Ibuprofen-Partikeln mit verbesserten Fließeigenschaften, insbesondere zur Abfüllung in Kapseln oder zur Verpressung zu Tabletten.

Bekanntlich sind die Partikelaröße und die Kristallform entscheidende Parameter für die pharmazeutisch-technologischen Eigenschaften von racemischem bzw. optisch reinem Ibuprofen. Ebenso ist bekannt, daß das auf verschiedenen Reaktionswegen gewonnene Ibuprofen nadelförmige Kristalle aufweist, die sehr schlechte Fließ- bzw. Rieseleigenschaften aufweisen. Aus diesen Gründen kommt es zu Schwierigkeiten bei der galenischen Verarbeitung, z.B. bei der Verpressung zu Tabletten oder bei der Kapselherstellung. Man hat versucht, diese Schwierigkeiten dadurch zu überwinden, daß racemisches Ibuprofen durch Umkristallisation aufgearbeitet wird (EP-A 120.587, WO 90/03782, WO 92/08686), oder durch einen Schmelzvorgang aufgearbeitet wird (EP-A 362.728). Die erstgenannte Art der Aufarbeitung bedingt die Verwendung organischer Lösungsmittel, was aus Umweltgründen häufig problematisch ist. Dieser Nachteil ist zwar bei der zweitgenannten Aufarbeitungsart vermieden, jedoch erfordert die dort beschriebene Vorgangsweise einen erheblichen apparativen Aufwand, denn das Racemat wird aufgeschmolzen und anschließend auf einer Kontaktfläche abgekühlt. Es werden schuppenartige Gebilde erhalten, die unter speziellen Mahlbedingungen zerkleinert werden müssen. Der dadurch bedingte apparative Aufwand ist zu groß, um ökonomisch arbeiten zu können.

Hinzu kommt, daß S(+)-Ibuprofen, dessen pharmazeutische Wirksamkeit jene des Racemats weit übersteigt, nicht nur einen im Vergleich zum Racemat (75 - 78°C) wesentlich niedrigen Schmelzpunkt (50-54°C) hat, sondern auch gänzlich andere physikalische Eigenschaften, z.B. ein anderes Lösungsverhalten in üblichen Lösungsmitteln, so daß die Durchführung der oben genannten Verfahren für S(+)-Ibuprofen auch aus diesen Gründen nicht möglich ist.

Die Erfindung setzt sich zur Aufgabe, ein Verfahren zur Herstellung von S(+)-Ibuprofen-Partikeln mit verbesserten Fließeigenschaften, insbesondere zur Abfüllung in Kapseln oder zur Verpressung zu Tabletten, zu schaffen, welches ökonomisch arbeitet und daher in großem Maßstab wirtschaftlich und ohne wesentliche Verwendung organischer Lösungsmittel und somit ohne Umweltbelastung durchführbar ist, einen geringen apparativen Aufwand benötigt und auch ein kontinuierliches Verfahren ermöglicht. Die Erfindung löst diese Aufgabe dadurch, daß grobkristallines S(+)-Ibuprofen aufgeschmolzen wird und sodann im geschmolzenen Zustand in einem Nichtlösemittel, vorzugsweise kaltem Wasser, fein verteilt und zur Erzielung einer feinkristallinen Primärstruktur abgeschreckt wird, worauf das in Agglomeraten als Sekundärstruktur anfallende Produkt abfiltriert und getrocknet wird. Der plötzliche Temperatursturz, welcher durch die Einwirkung des Nichtlösemittels auf das geschmolzene S(+)-Ibuprofen erfolgt, bewirkt eine Erstarrung des geschmolzenen Wirkstoffes und ein Auskristallisieren in einer Partikelform, die überraschender Weise einer Granulatform stark ähnelt. Vor allem ist hiebei überraschend, daß sich eine Primärstruktur in Form unregelmäßig geformter Kristallite ergibt, deren Verhältnis von Länge zu Breite nicht über etwa 1:2 hinaus geht. Diese Kristallite agglomerieren zu einer Sekundärstruktur in Form von Körnchen im allgemeinen mit einem Durchmesser von unter 1 mm, die im wesentlichen sphäroid und daher gut rieselfähig sind. Derartige Teilchen können direkt mit üblichen Hilfsmitteln zu Tabletten verpreßt werden bzw. es kann eine exakte Dosierung für die Herstellung von Tabletten, Kapseln oder anderen galenischen Formen erfolgen. Eine Zerkleinerung der erhaltenen Körnchen ist in der Regel nicht erforderlich, nur dort, wo es auf eine im wesentlichen einheitliche Körnchengröße ankommt, kann gegebenenfalls eine Klassierung, z.B. durch Siebung, erforderlich sein.

Das erfindungsgemäße Verfahren läßt sich ohne organische Lösungsmittel durchführen und vermeidet damit jedwede Umweltprobleme. Es hat eine hohe Ausbeute, die über 99% liegen kann und hat im Vergleich zu Verfahren, die mit dem Einsatz organischer Lösungsmittel arbeiten, einen höheren Durchsatz. Der apparative und zeitliche Aufwand ist im Vergleich zu allen oben erwähnten Verfahren wesentlich geringer. Weiters besteht die Möglichkeit, die Produkt-Korngröße durch variable Prozeßparameter zu beeinflussen, worauf später noch näher eingegangen werden wird. Das erfindungsgemäße Verfahren läßt sich sowohl chargenweise als auch kontinuierlich durchführen und kann unmittelbar nach der üblichen Herstellung von optisch reinem S(+)-Ibuprofen aus Racemat bzw. nach dem dort üblichen Endreinigungsschritt mittels Auskristallisieren aus Hexan erfolgen. Handelsübliches S(+)-Ibuprofen weist in der Regel einen Mindestgehalt von etwa 98% auf, unwesentliche Anteile an R(-)-Ibuprofen wirken jedoch beim erfindungsgemäßen Verfahren nicht störend. Die Vorteile des erfindungsgemäßen Verfahrens und des dadurch hergestellten Produktes zeigen sich umso deutlicher, je reiner die Ausgangssubstanz ist.

Weiters ist beim erfindungsgemäßen Verfahren vorteilhaft, daß das erhaltene Produkt es ermöglicht, die Hilfsstoffmengen für die galenische Weiterverarbeitung zu reduzieren, was eine größere Vielfalt in den galenischen Möglichkeiten zur Folge hat.

Der Grund, warum beim erfindungsgemäßen Verfahren granulatähnliche Teilchen erhalten werden, deren Verhältnis von Länge zu Breite der aus Kristalliten aufgebauten Teilchen nicht wesentlich von 1:1 abweicht, ist noch nicht erforscht. Es kann vermutet werden, daß die auf das Nichtlösemittel ausgeübte starke Scherung ein wesentlicher Faktor ist, denn das Ausmaß der Scherung bestimmt entscheidend die Korngröße und damit die Dissolutionsgeschwindigkeit des anfallenden S(+)-Ibuprofen-Agglomerats, so daß über das Ausmaß der Scherung eine Einstellung auf jeweils erforderliche Parameter möglich ist, ebenso wie eine Bewegung des Nichtlösemittels zwecks rascher Verteilung des zugesetzten geschmolzenen S(+)-Ibuprofens. Vorzugsweise wird daher beim erfindungsgemäßen Verfahren so vorgegangen, daß das geschmolzene S(+)-Ibuprofen dem Nichtlösemittel unter starkem Rühren zugegeben wird. Wie erwähnt, bestimmen der Einsatz einer starken Rührung sowie das Ausmaß der Scherung entscheidend die Korngröße des anfallenden S(+)-Ibuprofens. Günstig ist der Einsatz hochtouriger Werkzeuge für den Rührvorgang (z.B. der Einsatz eines Ultra-Turrax oder eines Turborührers), da auf diese Weise geringe Korngrößen des Agglomerats erzielbar sind und eine anschließende mechanische Zerkleinerung des erhaltenen Produktes erspart werden kann. Die Aufschmelztemperatur beträgt im Rahmen des erfindungsgemäßen Verfahrens bis zu 62°C. Bei dieser Temperatur ist S(+)-Ibuprofen als sogenannte Bulk Ware vollständig geschmolzen.

Die abschließende Trocknung des auskristallisierten Produktes erfolgt bei maximal 40°C, um eine neuerliche Aufschmelzung des S(+)-Ibuprofens zu vermeiden. Die Trocknung erfolgt zweckmäßig im Hordenschrank oder unter Vakuum. Die Vakuumtrocknung ist besonders vorteilhaft, da mit ihr die neuerliche Aufschmelzung leichter vermeidbar ist.

Bei der Auswertung der durchgeführten Untersuchungen stellte sich heraus, daß die eingesetzte Menge des Nichtlösemittels in Gewichtsprozent zweckmäßig der 3- bis 7-fachen Menge in Gewichtsprozent des eingesetzten S(+)-Ibuprofens beträgt, vorzugsweise der 5-fachen Menge. Eine wesentliche Erhöhung des Wasseranteils über diesen bevorzugten Wert verbessert die Produkteigenschaften nicht. Diese geringe Menge an einzusetzendem kaltem Nichtlösemittel ermöglicht größere Chargenansätze als bei Verfahren mit Einsatz vor organischen Lösungsmitteln. Die Verwendung von kaltem Wasser als Nichtlösemittel ist am zweckmäßigsten, es können jedoch auch andere bekannte Nichtlösemittel für den Abschreckvorgang verwendet werden, z.B. Mischungen von Wasser mit einem Anteil von wenigen Prozenten an organischer Flüssigkeit,wie Methanol, Äthanol usw., wobei die Verfahrensführung, insbesondere die Temperatur des eingesetzten Nichtlösemittels und bzw. oder die Menge des eingesetzten Wirkstoffes, so zu wählen sind, daß keine wesentliche Lösung des Wirkstoffes auftritt, d.h., daß bei den Prozeßbedingungen die Nichtlösekraft der Abschreckmittelmischung auf das Ibuprofen erhalten bleibt bzw. nicht wesentlich beeinträchtigt wird. Der Ausdruck "Nichtlösemittel" soll bedeuten, daß die Lösung unwesentlicher Anteile des Wirkstoffes zulässig ist.

Durch Einsatz verschiedener Hilfsstoffe, z.B. Zerfallhilfsmittel oder Bindemittel, lassen sich die Prozeßparameter der Galenik beeinflussen. Im Zuge der Untersuchungen hat es sich überraschender Weise gezeigt, daß unter Verwendung des in der erfindungsgemäßen Weise hergestellten S(+)-Ibuprofens nicht nur die direkte Verpressung schnell freisetzender Tabletten problemlos möglich war, also ohne aufwendige Granulierungsschritte, sondern auch die Herstellung von oralen Darreichungsformen mit verzögerter Wirkstofffreigabe, z.B. von Retardtabletten. Es kann vermutet werden, daß die feinkristalline Form der erhaltenen Kristallite bei der Agglomerierung zu den sphäroiden Teilchen der Sekundärstruktur eine dichtere Packung dieser Teilchen zur Folge hat, so daß die Löslichkeit der Wirkstoffteilchen durch Verringerung der freien Oberfläche herabgesetzt wird. Dadurch ergibt sich der Vorteil, daß keine gesonderte Matrix erforderlich ist, um eine Retardwirkung der galenischen Produkte zu erzielen. Auch dadurch wird die Reichhaltigkeit der galenischen Möglichkeiten vergrößert.

Gegebenenfalls können der Schmelze des S(+)-Ibuprofens Zerfallhilfsmittel, z.B. quervernetzte Carboxymethylcellulose, quervernetztes Polyvinylpyrrolidon oder mikrokristalline Cellulose zugesetzt werden, um eine schnellere Dissolution zwecks schnellerer Wirkstofffreisetzung zu erzielen. Ebenso können übliche Bindemittel, z.B. Cellulosederivate, insbesondere Hydroxypropylmethylcellulose Verwendung finden.

Im folgenden wird das erfindungsgemäße Verfahren anhand einiger Beispiele beschrieben:

### Beispiel 1

100,0 g grobkristallines S(+)-Ibuprofen werden in einem Edelstahlgefäß mittels Heizplatte erwärmt. Bei 60°C Produkttemperatur ist der Wirkstoff vollständig geschmolzen.

500,0 g kaltes Wasser (Raumtemperatur, ca. 20°C) werden in einem geeigneten Gefäß (Becherglas) vorgelegt und mit einem Magnetrührer gerührt.

DieS(+)-Ibuprofenschmelze wird in einem Guß unter Fortsetzung des Rührens dem Wasser zugegeben. Nach kurzer Zeit bildet sich ein festes granulatförmiges Produkt, welches anschließend mittels geeignetem Filter von der wäßrigen Phase getrennt wird. Das Endprodukt wird 2 Stunden bei 40°C unter Vakuum getrocknet und abschließend durch ein 1,25 mm-Frewittsieb gegeben. (Die Endtemperatur des Wassers nach erfolgtem Erstarren der Schmelze betrug 27°C).

### Beispiel 2

100,0 g grobkristallines S(+)-Ibuprofen werden entsprechend Beispiel 1 in 500,0 g kaltem Wasser auskristallisiert. Anstatt eines Turborührers wird ein Ultra-Turrax-Rührer eingesetzt. Das entstandene Produkt wird 2 Stunden bei 40°C unter Vakuum getrocknet. Eine abschließende Zerkleinerung bzw. Siebung entsprechend Beispiel 1 ist nicht notwendig, da der Korndurchmesser unter 1,25 mm beträgt (Endtemperatur des Wassers: 27°C).

### Beispiel 3

120,0 g grobkristallines S(+)-Ibuprofen werden entsprechend Beispiel 1 und 2 geschmolzen und in der Schmelze 13,0 g Calcium-Carboxymethylcellulose unter Magnetrühren dispergiert. Die Dispersion wird anschließend in einem Guß zu 800 g kaltem Wasser unter Ultra-Turrax-Rühren zugegeben. Die weiteren Arbeitsschritte entsprechen Beispiel 2. Auch hier ist aufgrund der geringen Agglomeratgröße kein weiteres Zerkleinern und keine Siebung notwendig. (Endtemperatur des Wassers: 23°C)

### Beispiel 4

10 kg grobkristallines S(+) -Ibuprofen werden in einem Edelstahlgefäß mittels Heizplatte erwärmt. Bei 62°C Produkttemperatur ist der Wirkstoff vollständig geschmolzen.

50 kg Wasser (ca. 20°C) werden in einem 1501 Nirostafaß vorgelegt und mit einem Turborührer gerührt. Die S(+)-Ibuprofenschmelze wird in einem Guß zum Wasser gegeben, nach 20 Sekunden kristallisiert ein Produkt aus, das abfiltriert und bei 40°C 2 Stunden unter Vakuum getrocknet wird.

Anschließend wird das Endprodukt durch ein 1,25 mm-Frewitt-Sieb gegeben. (Endtemperatur des Wassers: 26°C)

### Beispiel 5

200 g grobkristallines S(+)-Ibuprofen werden in einem Edelstahlgefäß mittels Heizplatte erwärmt, bei ca. 60°C Produkttemperatur ist der Wirkstoff vollständig geschmolzen.

1000 g Wasser (ca. 20°C) werden in einem Becherglas vorgelegt und mit einem Ultra-Turrax gerührt.

Die S(+)-Ibuprofenschmelze gibt man anschließend kontinuierlich tropfenweise dazu, wobei ein feines Produkt auskristallisiert. Dieses wird bei 40°C 2 Stunden unter Vakuum getrocknet. Eine Zerkleinerung ist nicht notwendig. (Endtemperatur des Wassers: 28°C)

### Beispiel 6

200 g grobkristallines S(+)-Ibuprofen werden entsprechend Beispiel 5 geschmolzen und durch Zugabe zu kaltem Wasser auskristallisiert. Das Einbringen der Schmelze in das Nichtlösemittel erfolgte durch Einspritzung mittels einer beheizten Düse.

Das entstandene feine Produkt wird abfiltriert und bei 40°C unter Vakuum getrocknet. Eine weitere Zerkleinerung ist nicht notwendig. (Endtemperatur des Wassers: 27°C)

### Beispiel 7

300 g grobkistallines S(+)-Ibuprofen werden in einem Becherglas geschmolzen (bei 60°C Produkttemperatur) und mit einem Ultra-Turrax gerührt. Danach gibt man 1,5 kg Wasser (20°C) in einem Guß dazu; unter fortgeführtem Rühren fällt ein feines Produkt an, welches abfiltriert und bei 40°C 2 Stunden unter Vakuum getrocknet wird. Eine weitere Zerkleinerung ist nicht notwendig. (Endtemperatur des Wassers: 27°C)

### Beispielrezeptur für eine S(+)-Ibuprofentablette mit erfindungsgemäß aufgearbeitetem Wirkstoff (Angaben in mg/Tablette)

Für die Herstellung der Tablette werden folgende Bestandteile miteinander vermischt:

| | |
|---|---|
| S(+)-Ibuprofen hergestellt nach Beispiel 4 | 300,0 |
| mikrokristalline Cellulose | 96,0 |
| Calcium-Carboxymethylcellulose | 15,0 |
| Talcum | 15,0 |

Aus der Mischung wird die Tablette durch Direktverpressung hergestellt. Die fertige Tablette hat folgende Meßdaten:

| | |
|---|---|
| Tablettenmasse | 426 mg |
| Bruchfestigkeit | 11 kp |
| Form | rund, gewölbt |
| Durchmesser | 11 mm |
| Zerfall in H₂O (37°C) | max. 1:45 min |

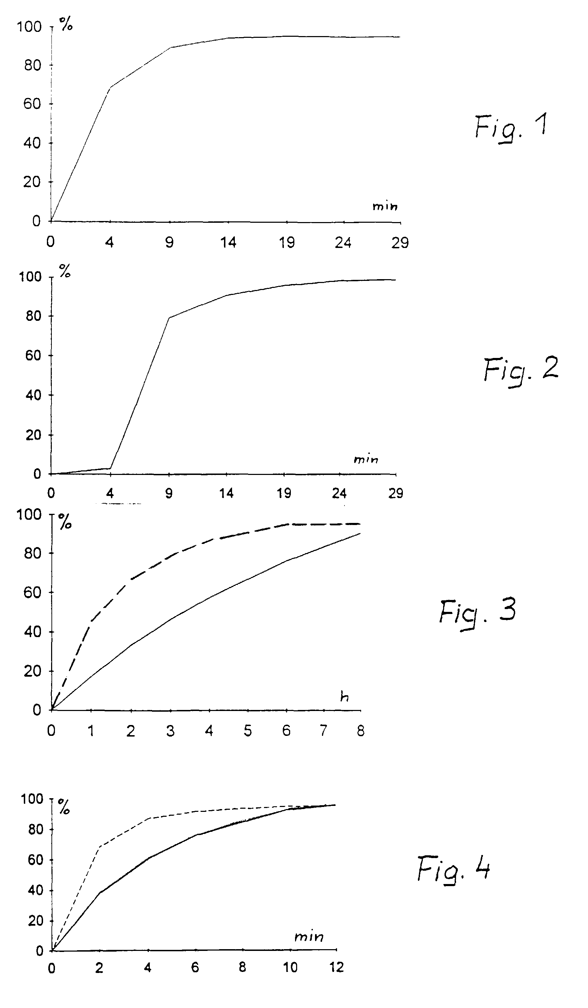

Die Freisetzung des Wirkstoffes aus so hergestellten Tabletten wurde untersucht. Als Medium diente Phosphatpuffer pH 7,2. Das Ergebnis ist in Form eines Schaubildes in Fig. 1 dargestellt, wobei auf der Ordinate der freigesetzte Wirkstoff in % und auf der Abszisse die Zeit in Minuten aufgetragen ist.

### Rezepturbeispiel für eine Hartgelatinekapsel mit erfindungsgemäß aufgearbeitetem Wirkstoff (Ansatz: 105 g)

Es wurden folgende Bestandteile vermischt:

| | |
|---|---|
| S(+)-Ibuprofen hergestellt nach Beispiel 1 | 100,0 g |
| Talcum | 5,0 g |

Die Mischung zeichnet sich durch sehr gute Rieseleigenschaften aus. Die Kapselabfüllung erfolgte auf einem üblichen Laborgerät.

Pro Kapsel wurden 190,5 mg Kapselfüllmasse verwendet, das entspricht 181,5 mg S(+)-Ibuprofen.

An so hergestellten Kapseln wurde die Freisetzung des Wirkstoffes untersucht. Die Ergebnisse sind im Schaubild nach Fig. 2 dargestellt, wobei auf der Ordinate der freigesetzte Wirkstoff in % und auf der Abszisse die Zeit in Minuten aufgetragen sind.

### Beispielrezeptur für eine retardierte S(+)-Ibuprofentablette mit erfindungsgemäß aufgearbeitetem Wirkstoff (Angaben in mg/Tablette)

Es wurden folgende Bestandteile vermischt:

| | |
|---|---|
| S(+)-Ibuprofen hergestellt nach Beispiel 4 | 400,0 |
| Hydroxypropylmethylcellulose | 40,0 |
| Montanglycolwachs | 40,0 |
| hochdisperses Siliciumdioxid | 2,7 |
| Talcum | 33,3 |

Aus der Mischung wurden durch Direktverpressung Tabletten hergestellt, die folgende Eigenschaften aufwiesen:

| | |
|---|---|
| Tablettenmasse | 516,0 mg |
| Bruchfestigkeit | 9 kp |
| Form | rund, gewölbt |
| Durchmesser | 11 mm |

Die so hergestellten Tabletten wurden hinsichtlich der Freisetzung des Wirkstoffes untersucht, wobei als Medium Phosphatpuffer pH 7,2 verwendet wurde. Unter gleichen Bedingungen wurden identisch rezeptierte Tabletten mit grobkristallinem, in der üblichen Weise hergestellten Wirkstoff untersucht. Die Ergebnisse sind im Diagramm nach Fig. 3 enthalten, in welchem auf der Ordinate der freigesetzte Wirkstoff in % und auf der Abszisse die Zeit in Stunden angegeben ist. Die mit dem erfindungsgemäß aufgearbeiteten Wirkstoff hergestellten Tabletten ergaben den mit vollen Linien dargestellten Freisetzungsverlauf, wogegen die mit dem grobkristallinen Wirkstoff hergestellten Vergleichstabletten den mit strichlierten Linien dargestellten Freisetzungsverlauf ergaben. Es ist ersichtlich, daß das Retardverhalten der den erfindungsgemäß aufgearbeiteten Wirkstoff enthaltenden Tabletten wesentlich günstiger war.

### Dissolutionsverhalten von S(+)-Ibuprofen

Es wurde folgende Versuchsanordnung verwendet:
400 mg S(+)-Ibuprofen wurden genau abgewogen und in 900 ml temperiertem Phosphatpuffer (37°C) pH 7,2 gegeben. Nach 2,4,6,8,10 und 12 min erfolgte jeweils ein Probenzug und anschließend die Bestimmung des aufgelösten Wirkstoffes. Die Dissolutionsversuche wurden an einem üblichen Testgerät mittels Paddle-Methode durchgeführt (6-fach-Bestimmung).

Es wurden folgende Ergebnisse erhalten:

Die Auswertung der Untersuchungen ergab, daß erfindungsgemäß aufgearbeitetes S(+)-Ibuprofen in den ersten Minuten etwas langsamer als grobkristalliner Wirkstoff im Medium gelöst wird. Nach etwa 10 Minuten ist sowohl das grobkristalline als auch das erfindungsgemäß aufgearbeitete S(+)-Ibuprofen vollständig gelöst. Wahrscheinliche Ursache dieser verlangsamten Dissolutionsverhaltens ist die Zusammenballung der Kristallite zu größeren Agglomeraten, sodaß vergleichsweise eine geringe Oberfläche des Wirkstoffes mit dem Medium in Kontakt ist.

Die Ergebnisse sind im Schaubild nach Fig. 4 dargestellt, in welchem auf der Ordinate der gelöste Wirkstoff in % und auf der Abszisse die Zeit in Minuten aufgetragen ist. Der mit vollen Linien dargestellte Verlauf entspricht erfindungsgemäß aufgearbeitetem S(+)-Ibuprofen, wogegen der mit strichlierten Linien dargestellte Verlauf grobkristallinem, in herkömmlicher Weise hergestellten S(+)-Ibuprofen entspricht.

### Strukturcharakterisierung von S(+)-Ibuprofen

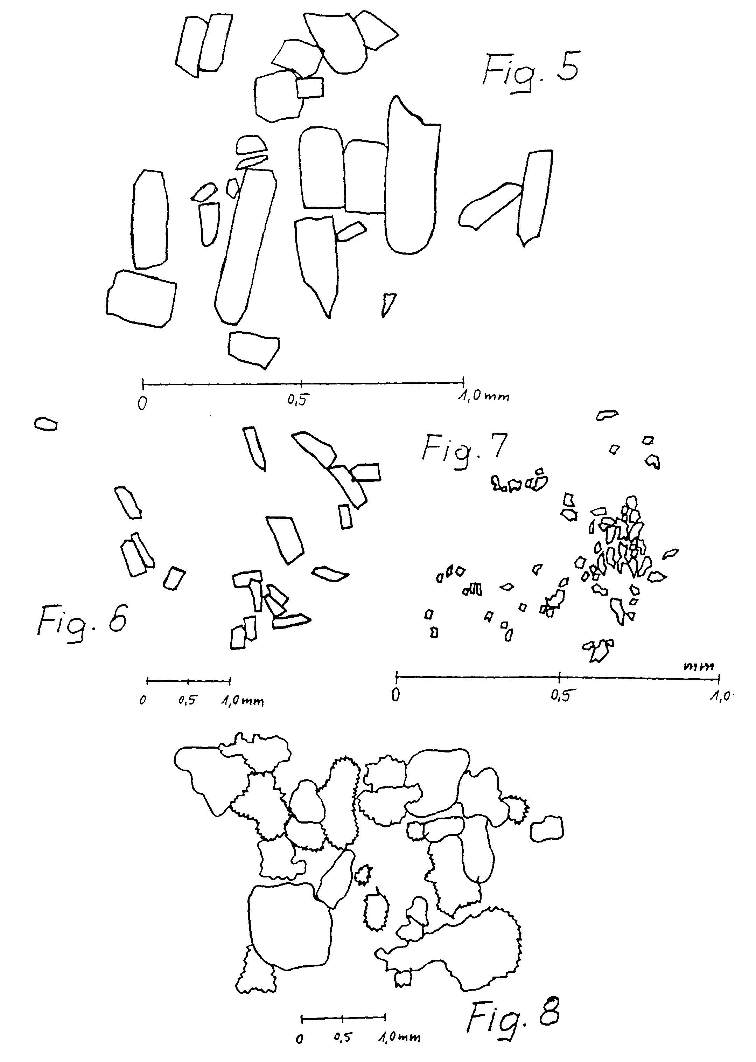

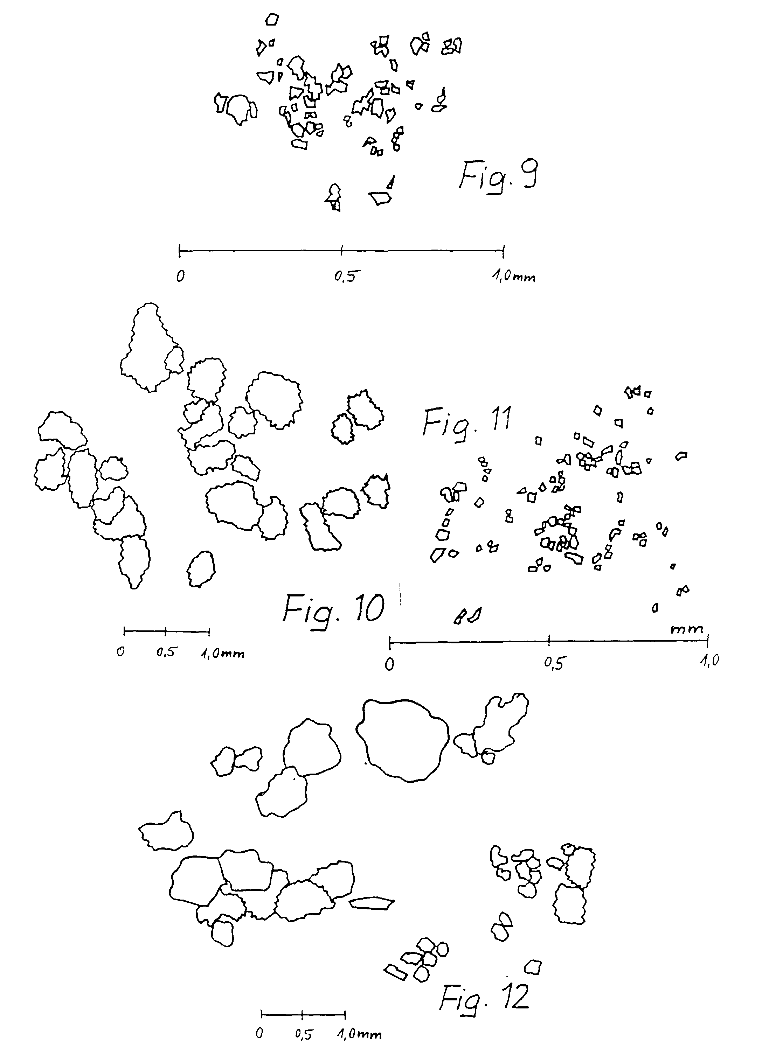

Die Strukturen von in erfindungsgemäßer Weise aufgearbeitetem S(+)-Ibuprofen und von in herkömmlicher Weise hergestelltem S(+)-Ibuprofen wurden unter dem Mikroskop untersucht. Die Ergebnisse sind in den Fig. 5 bis 12 dargestellt, wobei in den Fig. 5,7,9 und 11 jeweils die Primärstruktur in etwa 80-facher Vergrößerung dargestellt ist, in den Fig. 6,8,10 und 12 die Sekundärstruktur in etwa 20-facher Vergrößerung. Es ergaben sich:
1. Für grobkristallines, in üblicherweise aufbereitetes S(+)-Ibuprofen: Die Primärstruktur (Fig.5) entspricht der Sekundärstruktur (Fig.6). Es handelt sich um glasartig durchsichtige Kristalle mit säulenförmigem Aussehen, die eine glatte Oberfläche aufweisen. Das Material enthält relativ viel Splittermaterial. Die Länge der einzelnen Kristalle beträgt bis zu 500 pm, die Breite bis zu 150 pm, das Verhältnis Länge zu Breite im Durchschnitt ca. 1 : 3.
2. In erfindungsgemäßer Weise nach Beispiel 1 mittels eines Magnetrührers aufgearbeitetes S(+)-Ibuprofen:
   Als Primärstruktur (Fig.7) werden unregelmäßig geformte sphäroide Kristallite mit relativ einheitlicher Größe erhalten. Die Länge dieser Kristalle beträgt bis zu 60µm, die Breite bis zu 30µm, das Verhältnis Länge zu Breite im Durchschnitt 1:1 bis 1:2.
   Als Sekundärstruktur (Fig.8) ergaben sich Kristallitagglomerate mit zum Teil glatten Oberflächen. Zum Teil sind die Einzelkristallite erkennbar. Der Durchmesser der Agglomerate schwankt stark und beträgt bis etwa 1,5 mm.
3. In erfindungsgemäßer Weise nach Beispiel 2 mittels eines Ultra--Turrax aufgearbeitetes S(+)-Ibuprofen:
   Die Primärstruktur (Fig.9) zeigt unregelmäßig geformte sphäroide Kristallite mit relativ einheitlicher Größe. Die Länge beträgt bis zu 50 um, die Breite bis zu 20 µm, das Verhältnis Länge zu Breite im Durchschnitt ca. 1:1.
   Als Sekundärstruktur (Fig.10) ergab sich eine Kristallitagglomeration mit stark strukturierter Oberfläche. Der Durchmesser der Agglomerate schwankt relativ gering, er beträgt bis 1,0 mm.
4. In erfindungsgemäßer Weise nach Beispiel 3 mit Calcium-Carboxymethylcellulose als Hilfsstoff aufgearbeitetes S(+)-Ibuprofen:
   Als Primärstruktur (Fig.11) ergaben sich unregelmäßig geformte sphäroide Kristallite mit relativ einheitlicher Größe. Die Länge dieser Kristallite betrug bis zu 50 µm, die Breite bis zu 30 µm, das Verhältnis Länge zu Breite im Durchschnitt ca. 1 : 1.
   Als Sekundärstruktur (Fig.12) ergab sich eine nur zum Teil ausgeprägte Kristallitagglomeration mit vielen Einzelkristalliten. Glatte glänzende Oberflächen herrschten vor. Der Agglomeratdurchmesser betrug bis 1,5 mm.

### Identifizierung von erfindungsgemäß aufgearbeitetem S(+)-Ibuprofen in festen Arzneiformen (Tabletten, Dragees, Kapseln)

Die Unterschiede in Kristallform und -größe von herkömmlichen grobkristallinem und erfindungsgemäßem S(+)-Ibuprofen sind so gravierend, daß (abhängig von den eingesetzten Hilfsstoffen) eine relativ sichere Identifizierung der Wirkstoff-Genese möglich ist. In der Regel wird der Gehalt an S(+)-Ibuprofen immer sehr hoch sein, sodaß lediglich eine Beurteilung des Hauptbestandteils der Arzneiform notwendig ist. Dazu wird die Tablette bzw. das Dragee bzw. der Inhalt einer Kapsel mittels Mörser und Pistill schonend verrieben und das entstandene Pulver unter einem Mikroskop betrachtet.

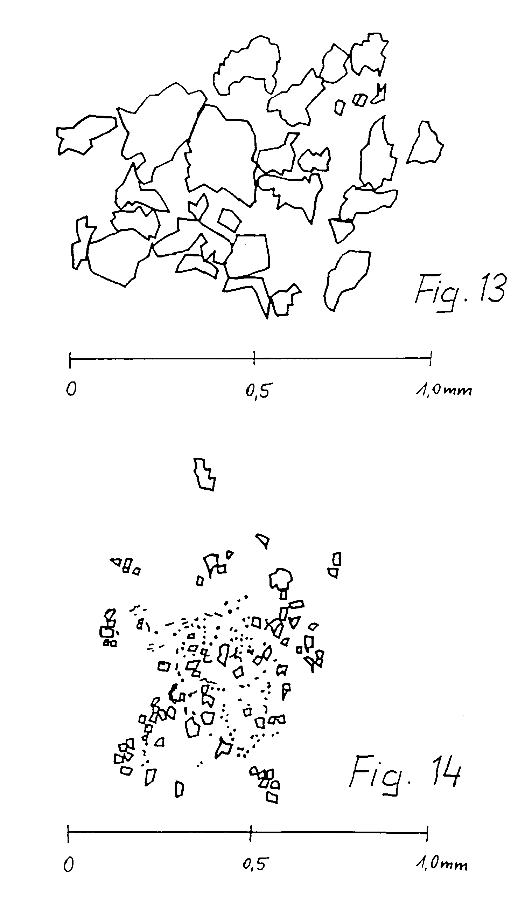

Die Fig.13 und 14 zeigen jeweils eine Probe mit in üblicher Weise aufgearbeitetem grobkristallinen Wirkstoff (Fig.13) und mit dem in erfindungsgemäßer Weise aufgearbeitetem Wirkstoff (Fig.14), jeweils in etwa 90-facher Vergrößerung.

## Patentansprüche

1. Verfahren zur Herstellung von S(+)-Ibuprofen-Partikeln mit verbesserter Fließeigenschaft, insbesondere zur Abfüllung in Kapseln oder zur Verpressung in Tabletten, dadurch gekennzeichnet, daß grobkristallines S(+)-Ibuprofen aufgeschmolzen wird und sodann im geschmolzenen Zustand in einem Nichtlösemittel, vorzugsweise kaltem Wasser, fein verteilt und die S(+)-Ibuprofen-Schmelze dabei zur Erzielung einer feinkristallinen Primärstruktur abgeschreckt wird, worauf das in Agglomeraten als Sekundärstruktur anfallende Produkt abfiltriert und getrocknet wird.

2. Verfahren nach Anspruch 1, dadurch gekennzeichnet, daß das geschmolzene S(+)-Ibuprofen dem Nichtlösemittel unter starkem Rühren zugegeben wird.

3. Verfahren nach Anspruch 2, dadurch gekennzeichnet, daß das Rühren mittels hochtouriger Werkzeuge erfolgt.

4. Verfahren nach Anspruch 1,2 oder 3, dadurch gekennzeichnet, daß das geschmolzene S(+)-Ibuprofen dem Nichtlösemittel in einem Guß zugesetzt wird.

5. Verfahren nach Anspruch 1,2 oder 3, dadurch gekennzeichnet, daß das geschmolzene S(+)-Ibuprofen dem Nichtlösemittel durch Einspritzen mittels einer beheizten Düse zugegeben wird.

6. Verfahren nach einem der Ansprüche 1 bis 5, dadurch gekennzeichnet, daß die Aufschmelzungstemperatur bis zu 62°C beträgt.

7. Verfahren nach einem der Ansprüche 1 bis 6, dadurch gekennzeichnet, daß das auskristallisierte Produkt bei maximal 40°C getrocknet wird, vorzugsweise unter Vakuum oder im Hordenschrank.

8. Verfahren nach einem der Ansprüche 1 bis 7, dadurch gekennzeichnet, daß die eingesetzte Menge des Nichtlösemittels in Gewichtsprozent der 3- bis 7-fachen Menge in Gewichtsprozent des S(+)-Ibuprofens beträgt, vorzugsweise der 5-fachen Menge.

9. Verfahren nach einem der Ansprüche 1 bis 8, dadurch gekennzeichnet, daß als Nichtlösemittel Wasser mit einem geringen Prozentanteil an zumindest einer organischen Flüssigkeit, z.B. Methanol, Äthanol usw. eingesetzt wird, wobei die Verfahrensführung, insbesondere die Temperatur des eingesetzten Nichtlösemittels und bzw. oder die Menge des eingesetzten S(+)-Ibuprofens so gewählt werden, daß keine wesentliche Lösung des Wirkstoffes auftritt.

10. Verfahren nach einem der Ansprüche 1 bis 9, dadurch gekennzeichnet, daß der Schmelze des S(+)-Ibuprofens Zerfallhilfsmittel, z.B. quervernetzte Carboxymethylcellulose, quervernetztes Polyvinylpyrrolidon oder mikrokristalline Cellulose, und bzw. oder Bindemittel, z.B. Cellulosederivate, insbesondere Hydroxypropylmethylcellulose zugesetzt werden.

11. S(+)-Ibuprofen-Partikel, gekennzeichnet durch Kristallite mit einer feinkristallinen Primärstruktur, die zu Agglomeraten als Sekundärstruktur agglomeriert sind.

12. S(+)-Ibuprofen-Partikel, dadurch gekennzeichnet, daß sie gemäß einem Verfahren nach einem oder mehreren der Ansprüche 1 bis 10 hergestellt worden sind.

13. Verwendung von Ibuprofen S(+)-Partikeln nach Anspruch 12, gegebenenfalls unter Zusatz von Hilfs- und/oder Trägerstoffen, zur Herstellung von Arzneimittel-Darreichungsformen, insbesondere von direkt verpreßten Tabletten oder Hartgelatine-Kapseln.

14. Verwendung nach Anspruch 13, zur Herstellung, unter Zusatz von Tablettierungs-Hilfsmitteln, von Tabletten mit verzögerter Wirkstofffreigabe.

## Claims

1. A process for producing S(+)-ibuprofen particles with improved flow properties, in particular for being poured into capsules or compressed into tablets, characterised in that coarsely crystalline S(+)-ibuprofen is melted and then, in the molten state, finely dispersed in a non-solvent, preferably cold water, and the S(+)-ibuprofen melt is quenched in order to obtain a finely crystalline primary structure, whereupon the product, in the form of agglomerates as a secondary structure, is filtered off and dried.

2. A process according to claim 1, characterised in that the molten S(+)-ibuprofen is added to the non-solvent while being vigorously stirred.

3. A process according to claim 2, characterised in that stirring is carried out by means of high-speed appliances.

4. A process according to claim 1, 2 or 3, characterised in that the molten S(+)-ibuprofen is added to the non-solvent in one go.

5. A process according to claim 1, 2 or 3, characterised in that the molten S(+)-ibuprofen is added to the non-solvent by being sprayed in by means of a heated nozzle.

6. A process according to any one of claims 1 to 5, characterised in that the melting temperature is up to 62°C.

7. A process according to any one of claims 1 to 6, characterised in that the crystallised product is dried at a maximum of 40°C, preferably in a vacuum or in a tray cabinet.

8. A process according to any one of claims 1 to 7, characterised in that the amount of non-solvent used in percent by weight is 3 to 7 times the amount in percent by weight of the S(+)-ibuprofen, preferably 5 times the amount.

9. A process according to any one of claims 1 to 8, characterised in that water with a low percentage of at least one organic liquid, e.g. methanol, ethanol, etc., is used as the non-solvent, the process being conducted, in particular the temperature of the non-solvent used and/or the amount of the S(+)-ibuprofen used being selected so that there is no substantial dissolution of the active ingredient.

10. A process according to any one of claims 1 to 9, characterised in that disintegration aids, e.g. cross-linked carboxymethyl cellulose, cross-linked polyvinylpyrrolidone or microcrystalline cellulose, and/or binders, e.g. cellulose derivatives, in particular hydroxypropyl methyl cellulose, are added to the S(+)-ibuprofen melt.

11. S(+)-ibuprofen particles, characterised by crystallites with a finely crystalline primary structure, the crystallites being agglomerated to form agglomerates as a secondary structure.

12. S(+)-ibuprofen particles, characterised in that they are produced by a process according to one or more of claims 1 to 10.

13. Use of ibuprofen S(+)-particles according to claim 12, optionally with the addition of auxiliary and/or supporting agents, for the preparation of pharmaceutical dosage forms, in particular directly compressed tablets or hard gelatin capsules.

14. Use according to claim 13 for the preparation of tablets with delayed release of the active ingredient, with the addition of tabletting aids.

## Revendications

1. Procédé de production de particules d'ibuprofène S(+) avec une meilleure aptitude à l'écoulement, en particulier en vue de leur conditionnement en gélules ou de leur compression sous forme de comprimés, caractérisé en ce que de l'ibuprofène S(+) à gros cristaux est fondu, puis finement dispersé à l'état fondu dans un milieu non solvant, de préférence de l'eau froide, et en ce que la masse fondue d'ibuprofène S(+) est refroidie brusquement en vue d'obtenir une structure primaire à cristaux fins, à la suite de quoi le produit obtenu en tant que structure secondaire sous forme d'agglomérats est séparé par filtration puis séché.

2. Procédé selon la revendication 1, caractérisé en ce que l'ibuprofène S(+) fondu est ajouté au milieu non solvant sous forte agitation.

3. Procédé selon la revendication 2, caractérisé en ce que l'agitation est assurée par un appareil à haute vitesse de rotation.

4. Procédé selon la revendication 1, 2 ou 3, caractérisé en ce que l'ibuprofène S(+) fondu est ajouté au milieu non solvant en une seule fois.

5. Procédé selon la revendication 1, 2, ou 3, caractérisé en ce que l'ibuprofène S(+) fondu est ajouté au milieu non solvant par injection au moyen d'une buse chauffée.

6. Procédé selon l'une des revendications 1 à 5, caractérisé en ce que la température de fusion est inférieure ou égale à 62°C.

7. Procédé selon l'une des revendications 1 à 6, caractérisé en ce que le produit obtenu à l'état cristallin est séché à une température maximale de 40°C, de préférence sous vide ou dans un sécheur à claies.

8. Procédé selon l'une des revendications 1 à 7, caractérisé en ce que la quantité utilisée de milieu non solvant correspond, en pourcentage pondéral, à 3 à 7 fois la quantité, en pourcentage pondéral, d'ibuprofène S(+), de préférence à 5 fois ladite quantité.

9. Procédé selon l'une des revendications 1 à 8, caractérisé en ce que l'eau utilisée en tant que milieu non solvant est additionnée d'un faible pourcentage d'au moins un liquide organique, par exemple le méthanol, l'éthanol etc., le mode opératoire, en particulier la température du milieu non solvant utilisé et/ou la quantité d'ibuprofène S(+) mise en oeuvre, étant choisi de façon qu'il ne se produise aucune dissolution significative du principe actif.

10. Procédé selon l'une des revendications 1 à 9, caractérisé en ce qu'à la masse fondue d'ibuprofène S(+) sont ajoutés des adjuvants de désagrégation, par exemple de la cellulose carboxyméthylique réticulée, de la polyvinylpyrrolidone réticulée ou de la cellulose microcristalline, et/ou des agents liants, par exemple des dérivés cellulosiques, en particulier l'hydroxypropylméthylcellulose.

11. Particule d'ibuprofène S(+), caractérisée par des cristallites dotées d'une structure primaire à cristaux fins, qui sont agglomérées en agglomérats en tant que structure secondaire.

12. Particule d'ibuprofène S(+) caractérisée en ce qu'elle est produite suivant un procédé selon l'une au moins des revendications 1 à 10.

13. Utilisation de particules d'ibuprofène S(+) selon la revendication 12, éventuellement avec addition d'adjuvants et/ou d'agents supports, pour la production de formes galéniques du médicament, en particulier de comprimés directement compressés ou de gélules en gélatine dure.

14. Utilisation selon la revendication 13, pour la production, sous addition d'adjuvants de compression, de comprimés à libération différée du principe actif.
